Europäisches Patentamt

European Patent Office

Office européen des brevets

(11) Veröffentlichungsnummer: **0 157 229**
**B1**

(12) **EUROPÄISCHE PATENTSCHRIFT**

(45) Veröffentlichungstag der Patentschrift:
**10.06.87**

(21) Anmeldenummer: **85102739.1**

(22) Anmeldetag: **11.03.85**

(51) Int. Cl.⁴: **C 12 M 1/26**

(54) **Vorrichtung zur Prüfung der Luft auf ihren Keimgehalt.**

(30) Priorität: **17.03.84 DE 3410990**

(43) Veröffentlichungstag der Anmeldung:
**09.10.85 Patentblatt 85/41**

(45) Bekanntmachung des Hinweises auf die Patenterteilung:
**10.06.87 Patentblatt 87/24**

(84) Benannte Vertragsstaaten:
**AT BE CH DE FR GB IT LI LU NL SE**

(56) Entgegenhaltungen:
**DE-B-2 301 385**
**FR-A-2 086 984**
**FR-A-2 389 119**
**GB-A-804 586**
**US-A-2 894 877**
**US-A-3 127 329**

(73) Patentinhaber: **Biotest- Serum- Institut GmbH,**
**Flughafenstrasse 4, D-6000 Frankfurt 71 (DE)**

(72) Erfinder: **Hempel, Hans Dieter, Froschgraben,**
**D-8752 Mainaschaff (DE)**
Erfinder: **Merz, Dieter, Dr. Dipl.- Chem.,**
**Schweinfurter Strasse 56, D-6000 Frankfurt (DE)**

(74) Vertreter: **Beil, Hans Christoph, Dr., Beil, Wolff**
**und Beil Rechtsanwälte Adelonstrasse 58, D-6230**
**Frankfurt am Main 80 (DE)**

EP 0 157 229 B1

LIBER, STOCKHOLM 1987

## Beschreibung

Die Erfindung betrifft eine Vorrichtung zur Prüfung der Luft auf ihren Keimgehalt nach dem Oberbegriff des Anspruchs 1.

In den Bereichen der Human- und Tiermedizin, der pharmazeutischen und der Lebensmittelindustrie ist der Nachweis von Keimen in der Luft oft von entscheidender Bedeutung.

Es sind viele Geräte für diesen Zweck bekannt, die nach unterschiedlichen Systemen arbeiten, unterschiedlich zu handhaben sind und unterschiedliche Ergebnisse sowohl in qualitativer als auch in quantitativer Hinsicht liefern.

Eine Zusammenstellung einer Vielzahl derartiger Geräte findet sich in "Air sampling instruments for evaluation of atmospheric contaminants", 5. Auflage (1978), America conference of governmental industrial hygienics.

Zu den wesentlichen Systemen der dort beschriebenen Geräte gehören 1) der Anderson-Sampler (S. L-7 und 0-20), 2) das Sartorius Staub-Sammelgerät (S. L-19 und K-40), 3) die Slit-Sampler von Casella und New Brunswick (S. 0-12 und 0-16) und 4) die sogenannte Stöber-Zentrifuge (S. 0-8 und 0-9).

Die Geräte 1 bis 3 besitzen den Nachteil, daß sie einen Netzanschluß aufweisen, d.h. netzabhängig sind, hohes Gewicht aufweisen, somit unhandlich und schwer zu transportieren sind und durch ihr großes Volumen und ihre Komplexizität schwer steril zu halten sind.

Die Geräte 1 und 2 erfordern darüberhinaus Vorarbeiten, die im Reinraum durchzuführen sind, und die Geräte 1 und 3 arbeiten mit Nährmedium in Petrischalen, was ebenfalls ein Handhabungsnachteil ist. Keines dieser Geräte arbeitet nach dem Zentrifugalprinzip. Gerät 4) ist von den genannten Geräten das einzige, das nach dem Zentrifugalprinzip arbeitet. Dieses Gerät ist jedoch äußerst umständlich in der Handhabung und Bedienung und routinemäßig nicht anwendbar.

In der DE-PS 2 301 385 wird eine Vorrichtung zur Prüfung der Luft auf ihren Keimgehalt beschrieben, die nach dem Zentrifugalprinzip arbeitet und einen relativ großen Luftraum in relativ kurzer Zeit erfaßt, klein, handlich, tragbar und netzunabhängig ist. Außerdem arbeitet sie mit einer mit Nährboden beschichteten Trägerfolie, die sich sauber und leicht in das Gerät einschieben und aus demselben entfernen läßt. Diese Vorrichtung arbeitet mit einem hochtourig zu betreibenden Flügelrad mit steil stehenden Flügeln, das mittig in einem starren Zylinder angeordnet ist, wobei die von oben angesaugte Luft gegen eine mit Nährboden beschichtete Trägerfolie geschleudert wird, die sich starr an der Innenwand des Zylindermantels befindet. Luftein- und -austritt erfolgen von oben durch die gleiche Öffnung.

Trotz der aufgezeigten Vorteile dieses Gerätes war man jedoch bestrebt, die Abscheideffektivität und deren Gleichmäßigkeit über einen weiten Partikelgrößenbereich zu verbessern. Außerdem findet während des Betriebes vor dem Gerät eine starke Wirbelbildung statt (in der DE-PS 2 301 385, Spalte 4 als "Windhose" beschrieben), die in gewissen Fällen, beispielsweise in der Nähe eines Operationsfeldes, insbesondere bei Knochenoperationen, wegen des damit verbundenen Risikos der Keimübertragung, nicht erwünscht ist.

Und schließlich läßt sich mit diesem Gerät der Luftdurchsatz nicht genau und auch nicht mit einfachen Mitteln bestimmen.

Der Erfindung liegt die Aufgabe zugrunde, eine Vorrichtung zur Prüfung der Luft auf ihren Keimgehalt bereitzustellen, die alle Vorteile der Vorrichtung gemäß DE-PS 2 301 385 aufweist, d.h. klein, handlich, tragbar und netzunabhängig ist und einen großen Luftraum in kurzer Zeit erfaßt, bei der jedoch ein weiterer Partikelgrößenbereich mit fast gleicher Effektivität erfaßt wird, über der während des Betriebes keine oder nur geringe Wirbelbildung stattfindet, deren Luftdurchsatz mit einfachen Mitteln bestimmt werden kann und deren Abscheidewirkung genau getestet werden kann und somit für den Benutzer eine bekannte Größe darstellt.

Diese Aufgabe wird bei einer gattungsgemäßen Vorrichtung durch die kennzeichnenden Merkmale des Anspruchs 1 gelöst.

Man fand, daß folgende Merkmale erfindungswesentlich für die Erzielung der angestrebten Vorteile sind:
1.) Konstruktion des Flügelrades,
2.) Luftführung und
3.) Mitrotieren der Trägerfolie.

Das Flügelrad muß so konstruiert sein, daß es zum einen die nötige Luftleistung bringt, zum anderen aber möglichst wenig Teilchen auf ihm haften bleiben oder auf den Boden der Lüfterkammer geschleudert werden. Man fand, daß diese Voraussetzungen erfüllt sind, wenn die einzelnen Flügel nicht gerade und steil an der Achse des Flügelrades angebracht sind, sondern einen flachen Anstellwinkel zur Drehebene des Flügelrades aufweisen. Der Anstellwinkel sollte zweckmäßigerweise zwischen 15 und 40°, vorzugsweise 20 und 25° betragen. Mit einer solchen Flügelradkonstruktion ist es möglich, die Keime im oberen Partikelgrößenbereich mit sehr hoher Effektivität auf der Trägerfolie abzuscheiden. Man fand jedoch, daß nicht alle kleinen und kleinste Teilchen aufgrund ihres geringen Gewichts mit Hilfe der Zentrifugalkraft die Trägerfolie erreichen.

Um diese zu erfassen, spielt die Luftführung eine bedeutende Rolle. Sie muß so erfolgen, daß jedes Luftquantum gleich lang und im gleichen Abstand über die Trägerfolie streicht. Es soll möglichst kein Teil der Luft die Vorrichtung verlassen, ohne über die Agarfolie gestrichen zu sein.

Man fand, daß diese Voraussetzungen erfüllt sind, wenn zum einen ein sicher getrennter Luftein- und -austritt durch Eintrittsöffnungen von oben und Austrittsöffnungen nach unten erfolgt und zum anderen, wenn die Rotorinnenwand unterhalb der Lüfterkammer derart als Leitzylinder ausgebildet ist, daß sich zwischen Zylinderwandung und dem unteren Teil der Trägerfolie eine schmale Ringkammer bildet. In dieser Ringkammer streift die Luft in ruhiger Führung zwischen Außenwandung des Zylinders und Trägerfolie entlang, so daß sich dabei auch kleinste Schwebeteilchen auf dem Nährboden der Trägerfolie abscheiden können. Vor dem unteren Luftaustritt befindet sich außerdem eine Drosselvorrichtung, die dazu dient, der Luft in der Vorrichtung mehr Verweilzeit zu geben, wodurch die Wahrscheinlichkeit der Abscheidung der kleinen Partikel auf dem Nährmedium noch verbessert wird.

Als Drosselvorrichtung kann ein Drosselspalt zwischen Zylindermantel des Leitzylinders und dem äußeren Rotormantel dienen oder aber es können sich Bohrungen im äußeren Rotormantel oder an der Verbindungsstelle zwischen äußerem Rotormantel und Zylindermantel des Leitzylinders befinden.

Eine noch weitere Verbesserung der Abscheiderate wird dadurch erreicht, daß die Trägerfolie mitrotiert. Durch das Mitrotieren wird eine Wirbelbildung über der Nährbodenoberfläche durch die Bewegung des Flügelrades vermieden, so daß im Inneren des Rotors eine weniger turbulente Luftströmung herrscht und dadurch ein geordnetes Abscheiden kleiner Teilchen ermöglicht wird.

Der getrennte Luftein- und -austritt besitzt noch den weiteren Vorteil, daß über der Vorrichtung keine oder nur geringe Wirbelbildung stattfindet und daß der Luftdurchsatz des Gerätes mit relativ einfachen Mitteln sehr genau bestimmt werden kann und somit die Abscheidewirkung berechnet werden kann, so daß dem Benutzer ein Gerät mit einer definierten und bekannten Abscheidewirkung bereitgestellt wird.

Der Rotor sitzt auf einem vorzugsweise runden Gehäuse und wird durch einen in dem Gehäuse sich befindlichen Motor über eine Antriebswelle und eine Magnetkupplung angetrieben, welche ein leichtes Entfernen des Rotors vom Antrieb und dessen Sterilisation gestattet.

Der Motor ist netzunabhängig und wird durch Batterien oder Akkus betrieben. Vorzugsweise verwendet man wiederaufladbare Elemente. Der Batterie- oder Akkupack befindet sich vorzugsweise am Boden des Gehäuses, um die Standsicherheit des Gerätes zu verbessern.

In einer bevorzugten Ausführungsform befindet sich über den Batterien oder Akkus eine Steuerelektronik, mit deren Hilfe die Rotationsgeschwindigkeit unabhängig vom Ladezustand der Batterie oder Akkus weitgehend konstant gehalten wird. Sie signalisiert außerdem, wenn Batterie- oder Akkupack für einen ordnungsgemäßen Betrieb nicht mehr ausreichen. Ferner übt sie eine Bremswirkung auf den relativ schweren Rotor aus, der sich ohne dieselbe aufgrund seines Gewichtes nach dem Abschalten unverhältnismäßig lange weiter drehen würde.

Den oberen Abschluß des Gehäuses bildet ein Motorhalter mit der Motor/Tacho-Kombination.

Der Rotor selbst ist aus Sicherheitsgründen mit einer Schutzhaube abgedeckt.

Die mit Nährboden beschichtete Trägerfolie entspricht in ihrer Länge dem inneren Umfang des Rotormantels und in ihrer Breite der Höhe des Rotors und weist an einem Ende eine Lasche auf. Zum Zwecke des leichteren Auszählens der Keime, der verbesserten Agarhaftung und der leichteren Biegbarkeit der Folie ist sie vorzugsweise in einzelne Näpfchen unterteilt. Vor Inbetriebnahme wird die sterile Folie mit Hilfe eines Schlitzes in den Rotor eingeschoben, wobei die Lasche aus dem Schlitz herausragt. Sie entspricht im wesentlichen der in der DE-PS 2 301 385 beschriebenen Trägerfolie und ist dort im einzelnen genau beschrieben.

Nachstehende Zeichnungen dienen der weiteren Erläuterung der Erfindung.
Es zeigen
Fig. 1 eine Ausführungsform eines Rotors.
Fig. 2 eine andere Ausführungsform eines Rotors.
Fig. 3 Gehäuse mit Schutzhaube.

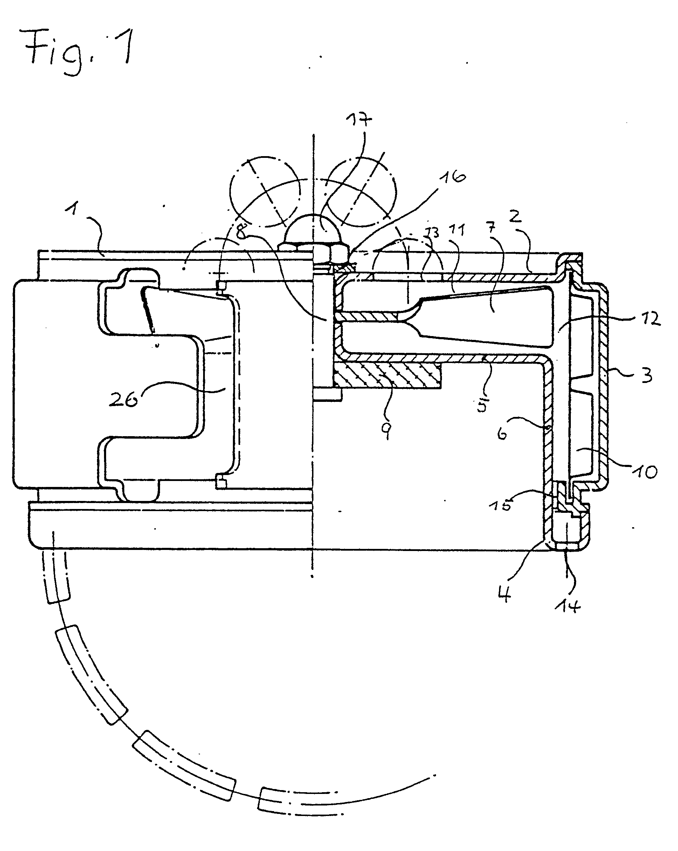

Fig. 1 zeigt den Rotor (1) mit Rotordeckel (2), Rotormantel (3) mit Leitzylinder (4), der aus Zylinderboden (5) und Zylindermantel (6) besteht. Rotordeckel (2) und Zylinderboden (5) bilden eine Lüfterkammer (11), in der sich das an einer Welle (8) befestigte Flügelrad (7) befindet. Die Höhe der Lüfterkammer beträgt etwa 1/3 bis 1/2 der Breite der Trägerfolie (10). Unter der Lüfterkammer (11) nahe der Welle (8) befindet sich die Magnetkupplung (9). Trägerfolie (10) läuft an der Innenwand des Rotormantels (3) entlang. Zylindermantel (6) befindet sich in geringem Abstand von der Trägerfolie (10) und bildet mit dieser zusammen eine Ringkammer (12). Die Ringkammer (12) besitzt eine Breite von etwa 1 - 5 mm, vorzugsweise 1 - 3 mm.

Unterhalb der Trägerfolie (10) wird aus Rotormantel (3) und Zylindermantel (6) ein Drosselspalt gebildet. Im Rotordeckel (2) befinden sich die Eintrittsöffnungen (13) und am unteren Ende des Leitzylinders (4) die Austrittsöffnungen (14). Hutmutter (17) dient dem Zweck, alle Rotorteile zusammenzuhalten. Anstelle einer Hutmutter kann jedoch jede andere geeignete Zusammenhaltevorrichtung verwendet werden, wie z.B. eine Niet-, Löt- oder Schweißverbindung.

Wesentlich ist nur, daß die Verbindung der erforderlichen Temperaturbelastung standhält.

Zum Zwecke des Keimfreimachens muß das Material Temperaturen von 120°C Wasserdampf und 180°C Heißluft standhalten. Unter der Hutmutter (17) befindet sich eine Scheibe (16), die die Kraft von Hutmutter (17) auf Rotordeckel (2) überträgt. Auch hier sind andere Lösungen des Übertragens möglich. Schlitz (26) dient zum Einschieben der Trägerfolie (10).

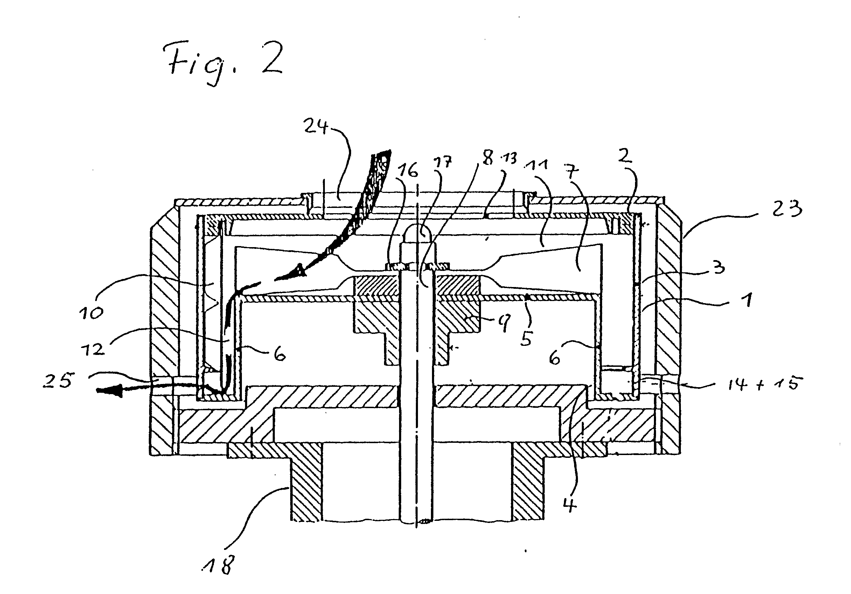

Fig. 2 zeigt eine weitere Ausführungsform eines Rotors (1), der in seinen Teilen (2) bis (13) und (16) bis (17) im wesentlichen mit Fig. 1 identisch ist, sich von Fig. 1 jedoch dadurch unterscheidet, daß die Drosselvorrichtung (15) und Austrittsöffnungen (14) identisch sind und durch Bohrungen im Rotormantel (3) gebildet werden und der Austritt der Luft seitlich am unteren Teil des Rotormantels (3) erfolgt. Außerdem wird hier die Position der Schutzhaube (23) gezeigt, die ebenfalls Eintrittsöffnungen (24) und Austrittsöffnungen (25) aufweist.

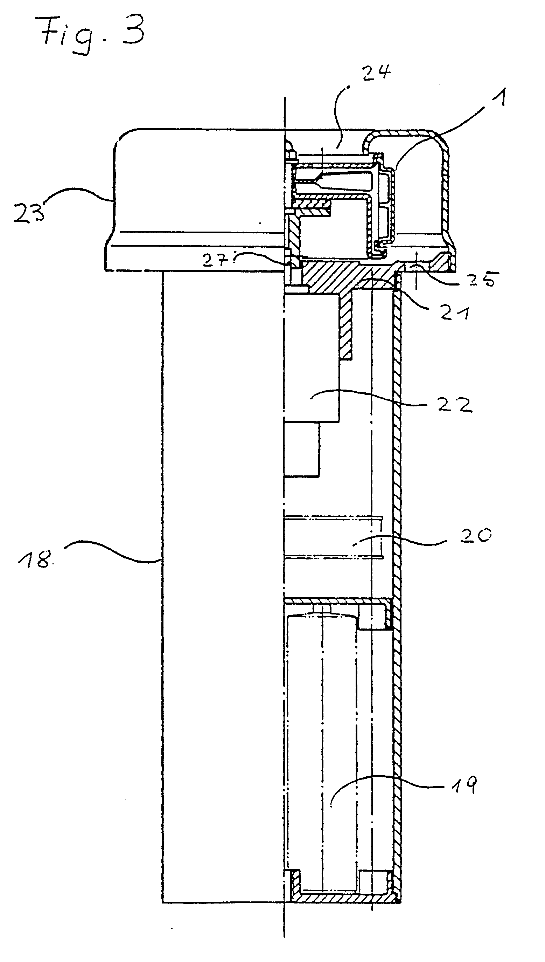

Fig. 3 zeigt das Gehäuse (18), das vorzugsweise rund ist, mit Batterie- oder Akkupack (19) am Boden. Darüber befindet sich die Steuerelektrode (20), auf der auch die Bedienungselemente angeordnet sein können. Den oberen Gehäuseabschluß bildet ein Motorhalter (21), der sowohl die Motor/Tacho-Kombination (22) mit Welle (27) hält, als auch die Schutzhaube (23) trägt. In dem seitlich über das Gehäuse (18) herausragenden Teil des Motorhalters (21) befinden sich Austrittsöffnungen (25), durch die die Luft bei Verwendung eines Rotors (1) gemäß Fig. 1 abströmen kann. Unter der Schutzhaube (23) mit Eintrittsöffnungen (24) befindet sich der Rotor (1).

Am Ort der Luftprobenentnahme wird in den Rotor (1) des sterilisierten Luftkeimsammlersystems die ebenfalls sterile mit Nährboden beschichtete Trägerfolie (10) von aussen so durch den Schlitz (26) eingeschoben, dass die offenliegende Nährbodenschicht nach innen zur Lüfterkammer (11) und Ringkammer (12) zu liegen kommt und der gesamte Innenumfang des Rotormantels (3) bedeckt wird. Dabei bleibt die Lasche ausserhalb des Rotors (1). Danach wird der Rotor (1) durch die Magnetkupplung (9) mit der Antriebswelle (27) des Motors (22) verbunden und darüber die Schutzhaube (23) angebracht.

Mit einem an der Steuerelektronik (20) angebrachten Schalter wird die Laufzeit eingestellt oder die Prüfluftmenge vorgewählt und danach die Starttaste bedient. Während des Betriebes tritt die Luft durch Eintrittsöffnungen (13) in die Lüfterkammer (11) mit Flügelrad (7) ein und wird nach aussen beschleunigt. Dabei scheiden sich bevorzugt die grösseren Partikel auf dem Nährmedium der Trägerfolie (10) ab. Die Luft und eventuell darin verbliebene kleinere Partikel bewegen sich jetzt in einer ruhigeren Strömung durch die Ringkammer (12) zwischen Trägerfolie (10) und Zylindermantel (6) nach unten. Die Verweilzeit der Luft wird durch die Drosselvorrichtung (15) verlängert. Der Luftaustritt erfolgt nach unten durch Austrittsöffnungen (14). Nach Ablauf der vorgewählten Laufzeit und Stillstand des Rotors (1) wird die Schutzhaube (23) abgenommen, die Trägerfolie (10) an ihrer Lasche aus dem Rotor (1) herausgezogen, in ihre Verpackung zurückgegeben, verschlossen und der Bebrütung zugeführt. Für weitere Luftproben ist lediglich jeweils eine neue, sterile, mit Nährmedium beschichtete Trägerfolie (10) in den Rotor (1) einzuschieben.

**Patentansprüche**

1. Vorrichtung zur Prüfung der Luft auf ihren Keimgehalt mit schnell rotierendem Flügelrad, mit dem die zu prüfende Luft gegen eine achsparallel angeordnete Nährbodenschicht strömt, wobei im oberen Teil eines Gehäuses ein netzunabhängig betriebener Motor über eine Antriebswelle und eine Magnetkupplung mit dem sich in einem Zylinder befindlichen Flügelrad verbunden ist und sich auf der Innenseite des Zylindermantels eine durch einen Schlitz von außen eingeschobene mit Nährmedium beschichtete Trägerfolie befindet, deren Länge dem inneren Umfang des Zylinders und deren Breite der Höhe des Zylinders entspricht und die eine aus dem Schlitz herausragende Lasche aufweist, dadurch gekennzeichnet, daß der Zylinder als Rotor (1) ausgebildet ist und der gesamte Rotor (1) durch den netzunabhängig betriebenen Motor (22) über Welle (27) und Magnetkupplung (9) und Welle (8) angetrieben wird, un sowohl das darin angeordnete Flügelrad (7) als auch die mit Nährmedium beschichtete Trägerfolie (10) in Umdrehung zu versetzen, der Rotor (1) einen inneren Leitzylinder (4) aufweist, dessen oben befindlicher Zylinderboden (5) zusammen mit dem Rotordeckel (2) oder einem Teil desselben eine Lüfterkammer (11) bildet, deren Höhe etwa 1/3 bis 1/2 der Breite der Trägerfolie (10) entspricht und in der das Flügelrad (7) angeordnet ist und dessen Zylindermantel (6) in geringem Abstand von der Trägerfolie (10) nach unten verläuft, wodurch zwischen Trägerfolie (10) und Zylindermantel (6) eine Ringkammer (12) gebildet wird, der Rotor (1) getrennte Luftein- und -austrittsöffnungen aufweist, wobei der Lufteintritt durch Eintrittsöffnungen (13) von oben und der Luftaustritt durch Austrittsöffnungen (14) nach unten erfolgt, denen eine Drosselvorrichtung (15) vorgeschaltet ist oder die selbst als Drosselvorrichtung ausgebildet sind, und der Anstellwinkel der Flügel des Flügelrades (7) zu dessen Drehebene relativ flach ist.

2. Vorrichtung nach Anspruch 1, dadurch gekennzeichnet, daß der Anstellwinkel zwischen 15° und 40°, vorzugsweise 20 bis 25° beträgt.

3. Vorrichtung nach Anspruch 1 oder 2, dadurch gekennzeichnet, daß der Rotor (1) mit einer Schutzhaube (23) ausgestattet ist.

4. Vorrichtung nach einem der vorstehenden Ansprüche, dadurch gekennzeichnet, daß die netzunabhängige Kraftquelle ein Batterie- oder Akkupack (19) ist und sich am Boden des Gehäuses (18) befindet.

5. Vorrichtung nach Anspruch 4, dadurch gekennzeichnet, daß die Kraftquelle wiederaufladbar ist.

6. Vorrichtung nach einem der vorstehenden Ansprüche, dadurch gekennzeichnet, daß sie mit einer Steuerelektronik (20) ausgestattet ist.

7. Vorrichtung nach einem der vorstehenden Ansprüche, dadurch gekennzeichnet, daß sie mit einem Motorhalter (21) ausgestattet ist.


**Revendications**

1. Dispositif pour l'évaluation de la concentration en germes dans l'air ayant une hélice tournant à grande vitesse qui fait circuler l'air à tester contre une couche de culture disposée parallèlement à l'axe de rotation, dans lequel un moteur fonctionnant independamment du réseau électrique de distribution est relié par un axe d'entraînement et un embrayage magnétique à une hélice située dans un cylindre à la partie supérieure d'un boîtier et dans lequel une feuille de support portant une couche d'un milieu de culture est glissée de l'extérieur par une fente du cylindre et est disposée sur la face périphérique interne de l'enveloppe cylindrique, ladite feuille ayant une longueur correspondant à la circonférence interne du cylindre et une largeur correspondant à la hauteur de celui-ci, et présentant une languette qui émerge de ladite fente, caractérisé en ce que le cylindre est conformé en rotor (1) et que l'ensemble du rotor (1) est entraîné par le moteur (22) indépendant du réseau par l'intermédiaire de l'axe (27) et de l'embrayage magnétique (9) et de l'axe (8), afin de mettre en rotation aussi bien l'hélice (7) disposée à l'intérieur dudit cylindre que la feuille support (10) revêtue du milieu de culture, le rotor (1) comportant un cylindre d'entraînement interne (4) dont le fond supérieur (5) coopère avec le couvercle (2) du rotor, ou une partie supérieure de celui-ci, pour former une chambre de ventilation (11) dont la hauteur correspond sensiblement à 1/3 à 1/2 de la largeur de la feuille (10), et dans laquelle est disposée l'hélice (7), et dont la paroi de cylindre (6) s'étend vers le bas à faible distance de la feuille (10) de manière à former entre cette feuille (10) et la paroi de cylindre (6) une chambre annulaire (12), en ce que le rotor (1) présente des ouvertures d'entrée et de sortie d'air séparées de sorte que l'entrée de l'air se fait par le haut par des ouvertures d'entrée (13) et la sortie de l'air se fait par le bas par des ouvertures de sortie (14), un dispositif d'étranglement (15) étant disposé en amont de ces dernières ou celles-ci formant elles-mêmes cet étranglement, et que l'angle d'incidence des pales de l'hélice (7) par rapport à son plan de rotation est relativement plat.

2. Dispositif selon la revendication 1, caractérisé en ce que l'angle d'incidence est compris entre 15° et 40° et de préférence entre 20° et 25°.

3. Dispositif selon l'une des revendications 1 ou 2 ci-dessus, caractérisé en ce que le rotor (1) est pourvu d'un capot de protection (23).

4. Dispositif selon l'une des revendications précédentes, caractérisé en ce que la source d'énergie indépendante du réseau est un bloc de piles ou d'accumulateurs (19) et est disposée sur le fond du boîtier (18).

5. Dispositif selon la revendication 4, caractérisé en ce que la source d'énergie est rechargeable.

6. Dispositif selon l'une des revendications précédentes, caractérisé en ce qu'il est équipé d'un ensemble électronique de commande (20).

7. Dispositif selon l'une des revendications précédentes, caractérisé en ce qu'il comporte un support moteur (21).


**Claims**

1. Device for testing air for microorganism content, having a rapidly rotating impeller by which the air to be tested is blown against an axially parallel layer of a culture medium, wherein in the top of a housing a motor driven independent of external power sources is connected to the impeller located in a cylinder through a driveshaft and a magnetic clutch, and on the inside surface of the jacket of the cylinder is a backing sheet to be inserted through a slot from the outside and coated with the culture medium, being as long as the inside circumference of the cylinder and as wide as the cylinder is high, and having a tab that projects out through the slot, <u>characterized in that</u> the cylinder forms a rotor (1) and the complete rotor (1) is powered by the motor (22) driven independent of external power through a shaft (27), and magnetic clutch (9) and shaft (8), so as to rotate the impeller (7) inside it as well as the culture medium-coated backing sheet (10), the rotor (1) has a channeling cylinder (4) inside it, the cylinder bottom (5) of which at the top in conjunction with the lid of the rotor (2) or at least part of it constitutes an impeller chamber (11) that is approximately 1/3 to 1/2 as high as the backing sheet (10) is wide, the chamber accomodates the impeller (7), and has a cylinder jacket (6) that extends down from the backing sheet (10) by a slight distance thereby defining, an annular chamber (12) between the backing sheet (10) and the cylinder jacket (6), the rotor (1) has separate air inlets and outlets, the air entering from above through the inlets (13) and leaving downward through the outlets (14), either the outlets constitute a constricted zone in themselves or there is a constricted zone positioned upstream of them, and the angle of the blades of the impeller (7) to its plane of rotation is relatively small.

2. Device according to Claim 1, characterized in that the angle is between 15 and 40°, preferably 20 and 25°.

3. Device according to Claim 1 or 2, characterized in that the rotor (1) is provided with a safety hood (23).

4. Device according to one of the preceding Claims, characterized in that the non-external power source is a battery or storage battery and is located at the base of the housing (18).

5. Device according to Claim 4, characterized in that the power source is rechargeable.

6. Device according to one of the preceding Claims, characterized in that it is provided with an electronic control (20).

7. Device according to one of the preceding Claims, characterized in that it is provided with a mount of the motor (21).

Fig. 1

Fig. 2

0 157 229

Fig. 3